# EUROPEAN PATENT SPECIFICATION

(11) **EP 1 294 867 B1**
(45) Date of publication and mention of the grant of the patent: **08.09.2004**
(21) Application number: 01941414.3
(22) Date of filing: 19.06.2001
(51) Int. Cl.: C12N 15/00, C12N 9/64

(54) **HUMAN SITE-1 PROTEASE PROMOTER**
MENSCHLICHER SITE-1 PROTEASE-SPEZIFISCHER PROMOTOR
PROMOTEUR DU SITE 1 DE PROTEASE HUMAINE

(30) Priority: 27.06.2000 SE 0002417
(43) Date of publication of application: 26.03.2003
(73) Proprietor: BIOVITRUM AB, 112 76 Stockholm (SE)
(72) Inventor: ABRAHMSEN Lars, S-168 58 Bromma (SE); EKBLOM, Jonas, San Diego, CA 92121 (US); FORSGREN, Margareta, S-116 32 Stockholm (SE); HÖRLING, Jan, S-181 42 Lidingö (SE); JOHANSSON, Per, S-164 71 Kista (SE)
(74) Representative: Höglund, Lars
(86) International application number: PCT/SE2001/001386
(87) International publication number: WO 2002/000873

(56) References cited:
- WO-A2-00/09677

### SEQUENCE LISTING

<110> Pharmacia AB
<120> Promoter Sequences
<130> 00130
<140>
   <141>
<160> 6
<170> PatentIn Ver. 2.1
<210> 1
   <211> 980
   <212> DNA
   <213> Homo sapiens
<400> 1
   ctccgcggcg aacacgcctg ggcactccat tcggggctgt ttactcccaa ctctcgcgag 60
   actgggcggc cgggccagcg aggcccacag ctgggagcct cagctccgcc gacccagcgt 120
   gccctgtctg tcccgcgctc ccggggcttg cgtgcgcgct ctggacgccg tgggcagcgg 180
   gaccacgccg ggaggatgga cgaaggtgct cgcgacattt gcggcggcgg gggccggtgg 240
   cagggtggaa gcggaggggc gtggccagcg agctgccagg cggcgagaac gcgctggggg 300
   aacccttggt ccgctctgcg cgtcgctcta ggatccccga aaaggagcac gggcgcgaaa 360
   gcggccaggc tgggccagga tctagaaaga ctgcctggcg caggctccct gcccccgcgg 420
   gcctgctgtc atggactcgt ggagagctcg cttcccgcgc ggacccttcc tgcaggggtc 480
   cacgtccagg caccggcggc tcggacaccc cacccccggc cgggcacctg ccctgggtgc 540
   cccttaaccc gggcggtagc tcgttaagat ggcgaagtgt ccggtccgga acacgcgaaa 600
   ccccaaatcc cgcctgcccg acctcctgac ccccggcccc acgggacgac agactgggcc 660
   tcccgacgcg cagcgcgctg ccgggacacc ggtgcgtgcg aaacggagga cctttgtaac 720
   gccacgtgtt tgctcttttt gaaaaaacaa gaataaatgt gttaaactgt ctgaaaagct 780
   tgccgcctaa aagatgtctg ggtgacttag atgctaggat cagtttgttt tcaatgtaaa 840
   tggaccagcc cggactccgt acggcactag caggggactg aaagcgtctt caggtactgc 900
   tggtgggcgg tgatgcgcta caggccgatc agacagtttt gtgtcttctg gaacttgaca 960
   ctgcaccacg gtaatgctga                                             980
<210> 2
   <211> 2469
   <212> DNA
   <213> Homo sapiens
<400> 2
   ttgagtctgt ctggaggctc cgggccagag cagggcgtat tgtttcactc ggtgaatgct 60
   catttcacgt aaagaaaacc aggcaacgga acaagctgcc ggagcgcgca gacccccgca 120
   gggccgcggt acaggcacgc tgtgtccaaa caagcgccgg aggccccgcg cccacctccc 180
   ccgacccggc ccggcccccg cagccctcgc ctcggggcct cggacgcaac cggcacacct 240
   gagcgagcgg gccgccaccg ctaggcggag cgggtcgggg aggccgcgcg cgggcggctg 300
   acgtacctgc gccgccggga gctcagggcc ggcgggcccg ggatgacggc gcctccgcgg 360
   cgaacacgcc tgggcactcc attcggggct gtttactccc aactctcgcg agactgggcg 420
   gccgggccag cgaggcccac agctgggagc ctcagctccg ccgacccagc gtgccctgtc 480
   tgtcccgcgc tcccggggct tgcgtgcgcg ctctggacgc cgtgggcagc gggaccacgc 540
   cgggaggatg gacgaaggtg ctcgcgacat ttgcggcggc gggggccggt ggcagggtgg 600
   aagcggaggg gcgtggccag cgagctgcca ggcggcgaga acgcgctggg ggaacccttg 660
   gtccgctctg cgcgtcgctc taggatcccc gaaaaggagc acgggcgcga aagcggccag 720
   gctgggccag gatctagaaa gactgcctgg cgcaggctcc ctgcccccgc gggcctgctg 780
   tcatggactc gtggagagct cgcttcccgc gcggaccctt cctgcagggg tccacgtcca 840
   ggcaccggcg gctcggacac cccacccccg gccgggcacc tgccctgggt gccccttaac 900
   ccgggcggta gctcgttaag atggcgaagt gtccggtccg gaacacgcga aaccccaaat 960
   cccgcctgcc cgacctcctg acccccggcc ccacgggacg acagactggg cctcccgacg 1020
   cgcagcgcgc tgccgggaca ccggtgcgtg cgaaacggag gacctttgta acgccacgtg 1080
   tttgctcttt ttgaaaaaac aagaataaat gtgttaaact gtctgaaaag cttgccgcct 1140
   aaaagatgtc tgggtgactt agatgctagg atcagtttgt tttcaatgta aatggaccag 1200
   cccggactcc gtacggcact agcaggggac tgaaagcgtc ttcaggtact gctggtgggc 1260
   ggtgatgcgc tacaggccga tcagacagtt ttgtgtcttc tggaacttga cactgcacca 1320
   cggtaatgct gaactgcacc aatattacag atcacagcgc atcatcttcc ttcaacatga 1380
   tttaacacag ttgacttaat atggtggata aatgtagaat cacaaattac cataccccac 1440
   ctcaggcttc tacttcgtaa ttttgagcag gttgtttaac ctctttgtac ctcagcttct 1500
   tcattacaaa aataggggta ctagccaggc ggggtggctc gcgcctgtaa tcccagcact 1560
   tggggaggcc gaggcagccg gatcacttga ggtcagaagt ttcagaccag cctggtcaac 1620
   atgggtgaaa cgccggctct accaaaaata taaaaactta gctgagtgtg gtagcgcatg 1680
   actgtaatcc cagcaactca ggaggctgag gcagagaatc gcttgaacct gggaggcgga 1740
   ggttgcagtg agctgagatc gtaccactgc actccagctt gggcgacaga gcgagactct 1800
   gccttaaaaa taaataaata atttttaaaa aaaatagggg tactaatatc taccttaaag 1860
   gatgagggtt aaattaagta cacacataag ccctagcgca gtggcttatg cctgtaatct 1920
   caacactttg ggagtctgtg gcgggaggat cacttgagcc caggagtttg agactagtct 1980
   gggcaacaga gacatgtctc tatagttgtg tttggttttg tttttaccag gtgtggtggt 2040
   gtgcacctgc agtcccagct actagggagg ctgaggtggg aggactgcct gagcccagga 2100
   ggtcgaggct gcagtgagcc atgattgtgc cactgcactc cagcctgggc aacacagcaa 2160
   gaccttgtct caaaaacaaa caaaaagcat actcataaag tgctcggctc ctatatgatt 2220
   caatatgtgg tggtggattc ttgaatcctt tcctgactca gatctcatac gattttctga 2280
   acttttggag aatccttgcc tctctgcatt tgcaaaccgt caaaggcact cccttctgcc 2340
   accacacaaa gcatttgatt ttaaacttga ctatgtcctt ctgttccaac tttaggtaaa 2400
   ttaatcttgg tcagggttct ctgaacagcc ctttagtcac tatgccattg aatacatggc 2460
   cctacagct                                                         2469
<210> 3
   <211> 4338
   <212> DNA
   <213> Homo sapiens
<220>
   <221> CDS
   <222> (497)..(3655)
<400> 3
cagggcacgc tgggtcggcg gagctgaggc tcccagctgt gggcctcgct ggcccggtcg 60
   cccagtctcg cgagagttgg gagtaaacag ccccgaatgg agtgcccagg cgtgttcgcc 120
   gcggaggcgc cgttatcccg ggcccgccgg ccctgagctc ccggcggcgc agattggctc 180
   acagtggttg attgatcaac cccattggac gttggttctg tggtacaaat ggagtacagg 240
   actcagtcgt cacggcctga gtgagagaag ccttatttcc aagatggaga agaagcggag 300
   aaagaaatga aagcctctct tcaggctgaa ccacaaaagg ccatgggatt taacttttat 360
   ttatgttggg caagactgta agatggctga tcagtaatgt tgcagctttt agctgaaaca 420
   aaaattcact tttaatcaag aagaaaaaag tgtgatttga atatatgcaa ttttatgatc 480
   atattcgctt gtgacc atg aag ctt gtc aac atc tgg ctg ctt ctg ctc gtg 532
   gtt ttg ctc tgt ggg aag aaa cat ctg ggc gac aga ctg gaa aag aaa   580
   tct ttt gaa aag gcc cca tgc cct ggc tgt tcc cac ctg act ttg aag   628
   gtg gaa ttc tca tca aca gtt gtg gaa tat gaa tat att gtg gct ttc   676
   aat gga tac ttt aca gcc aaa gct aga aat tca ttt att tca agt gcc   724
   ctg aag agc agt gaa gta gac aat tgg aga att ata cct cga aac aat   772
   cca tcc agt gac tac cct agt gat ttt gag gtg att cag ata aaa gaa   820
   aaa cag aaa gcg ggg ctg cta aca ctt gaa gat cat cca aac atc aaa   868
   cgg gtc acg ccc caa cga aaa gtc ttt cgt tcc ctc aag tat gct gaa   916
   tct gac ccc aca gta ccc tgc aat gaa acc cgg tgg agc cag aag tgg   964
   caa tca tca cgt ccc ctg cga aga gcc agc ctc tcc ctg ggc tct ggc   1012
   ttc tgg cat gct acg gga agg cat tcg agc aga cgg ctg ctg aga gcc   1060
   atc ccg cgc cag gtt gcc cag aca ctg cag gca gat gtg ctc tgg cag   1108
   atg gga tat aca ggt gct aat gta aga gtt gct gtt ttt gac act ggg   1156
   ctg agc gag aag cat ccc cac ttc aaa aat gtg aag gag aga acc aac   1204
   tgg acc aac gag cga acg ctg gac gat ggg ttg ggc cat ggc aca ttc   1252
   gtg gca ggt gtg ata gcc agc atg agg gag tgc caa gga ttt gct cca   1300
   gat gca gaa ctt cac att ttc agg gtc ttt acc aat aat cag gta tct   1348
   tac aca tct tgg ttt ttg gac gcc ttc aac tat gcc att tta aag aag   1396
   atc gac gtg tta aac ctc agc atc ggc ggc ccg gac ttc atg gat cat   1444
   ccg ttt gtt gac aag gtg tgg gaa tta aca gct aac aat gta atc atg   1492
   gtt tct gct att ggc aat gac gga cct ctt tat ggc act ctg aat aac   1540
   cct gct gat caa atg gat gtg att gga gta ggc ggc att gac ttt gaa   1588
   gat aac atc gcc cgc ttt tct tca agg gga atg act acc tgg gag cta   1636
   cca gga ggc tac ggt cgc atg aaa cct gac att gtc acc tat ggt gct   1684
   ggc gtg cgg ggt tct ggc gtg aaa ggg ggg tgc cgg gcc ctc tca ggg   1732
   acc agt gtt gct tct cca gtg gtt gca ggt gct gtc acc ttg tta gtg   1780
   agc aca gtc cag aag cgt gag ctg gtg aat ccc gcc agt atg aag cag   1828
   gcc ctg atc gcg tca gcc cgg agg ctc ccc ggg gtc aac atg ttt gag   1876
   caa ggc cac ggc aag ctc gat ctg ctc aga gcc tat cag atc ctc aac    1924
   agc tac aag cca cag gca agt ttg agc ccc agc tac ata gat ctg act    1972
   gag tgt ccc tac atg tgg ccc tac tgc tcc cag ccc atc tac tat gga    2020
   gga atg ccg aca gtt gtt aat gtc acc atc ctc aac ggc atg gga gtc    2068
   aca gga aga att gta gat aag cct gac tgg cag ccc tat ttg cca cag    2116
   aac gga gac aac att gaa gtt gcc ttc tcc tac tcc tcg gtc tta tgg    2164
   cct tgg tcg ggc tac ctg gcc atc tcc att tct gtg acc aag aaa gcg    2212
   gct tcc tgg gaa ggc att gct cag ggc cat gtc atg atc act gtg gct    2260
   tcc cca gca gag aca gag tca aaa aat ggt gca gaa cag act tca aca    2308
   gta aag ctc ccc att aag gtg aag ata att cct act ccc ccg cga agc    2356
   aag aga gtt ctc tgg gat cag tac cac aac ctc cgc tat cca cct ggc    2404
   tat ttc ccc agg gat aat tta agg atg aag aat gac cct tta gac tgg    2452
   aat ggt gat cac atc cac acc aat ttc agg gat atg tac cag cat ctg    2500
   aga agc atg ggc tac ttt gta gag gtc ctc ggg gcc ccc ttc acg tgt    2548
   ttt gat gcc agt cag tat ggc act ttg ctg atg gtg gac agt gag gag    2596
   gag tac ttc cct gaa gag atc gcc aag ctc cgg agg gac gtg gac aac    2644
   ggc ctc tcg ctc gtc atc ttc agt gac tgg tac aac act tct gtt atg    2692
   aga aaa gtg aag ttt tat gat gaa aac aca agg cag tgg tgg atg ccg    2740
   gat acc gga gga gct aac atc cca gct ctg aat gag ctg ctg tct gtg    2788
   tgg aac atg ggg ttc agc gat ggc ctg tat gaa ggg gag ttc acc ctg    2836
   gcc aac cat gac atg tat tat gcg tca ggg tgc agc atc gcg aag ttt    2884
   cca gaa gat ggc gtc gtg ata aca cag act ttc aag gac caa gga ttg    2932
   gag gtt tta aag cag gaa aca gca gtt gtt gaa aac gtc ccc att ttg    2980
   gga ctt tat cag att cca gct gag ggt gga ggc cgg att gta ctg tat    3028
   ggg gac tcc aat tgc ttg gat gac agt cac cga cag aag gac tgc ttt    3076
   tgg ctt ctg gat gcc ctc ctc cag tac aca tcg tat ggg gtg aca ccg    3124
   cct agc ctc agt cac tct ggg aac cgc cag cgc cct ccc agt gga gca    3172
   ggc tca gtc act cca gag agg atg gaa gga aac cat ctt cat cgg tac    3220
   tcc aag gtt ctg gag gcc cat ttg gga gac cca aaa cct cgg cct cta    3268
   cca gcc tgt cca cgc ttg tct tgg gcc aag cca cag cct tta aac gag    3316
   acg gcg ccc agt aac ctt tgg aaa cat cag aag cta ctc tcc att gac    3364
   ctg gac aag gtg gtg tta ccc aac ttt cga tcg aat cgc cct caa gtg    3412
   agg ccc ttg tcc cct gga gag agc ggc gcc tgg gac att cct gga ggg    3460
   atc atg cct ggc cgc tac aac cag gag gtg ggc cag acc att cct gtc    3508
   ttt gcc ttc ctg gga gcc atg gtg gtc ctg gcc ttc ttt gtg gta caa    3556
   atc aac aag gcc aag agc agg ccg aag cgg agg aag ccc agg gtg aag    3604
   cgc ccg cag ctc atg cag cag gtt cac ccg cca aag acc cct tcg gtg    3652
   tga ccggcagcct ggctgaccgt gagggccaga gagagccttc acggacggcg         3705
   ctggtgggtg agccgagctg tggtggcggc tggtttaaaa gggatccagt ttccagctgc 3765
   aggtttgtta gagtctgttc tacatgggcc tgccctcctg tgatgggcag aggctcctgg 3825
   tacatcgaga agattcctgt ggatcccgtc aggagggact tagtggctct gccgccagtg 3885
   agacttcccg ccggcagctg tgcgcaccaa agactcggga gaactggaaa ggctgtctgg 3945
   ggtcttctga ctgcagggga aggatgtact ttccaaacaa atgatacaac cctgaccaag 4005
   ctaaaagacg cttgttaaag gctattttct atatttattg ttgggaaaag tcactttaaa 4065
   gacttgtgct atttggaagc aaagctattt tttttgtcag tggaatgcag tttttttact 4125
   attccatcat gaggaacaac atagattcca tgatcttttt aatgacagta cagactgaga 4185
   tttgaaggaa acatgcacaa atctgtaaaa catagacctt cgctttattt ttgtaagtat 4245
   cacctgccac catgttttgt aatttgaggt cttgatttca ccattgtcgg tgaagaaaat 4305
   tttcaataaa tatgtattac ccgtctgaag ctt                              4338
<210> 4
   <211> 1052
   <212> PRT
   <213> Homo sapiens
<400> 4
<210> 5
   <211> 4198
   <212> DNA
   <213> Cricetulus griseus
<220>
   <221> CDS
   <222> (387) .. (3545)
<400> 5
   tgttcgcggc agaggcgccg ttcccccggg cccgccgacc tcgagcctga ggcggacgca 60
   ggtcggccct cagagtggtt tcttgggcat ccccactaga tttgggtctg tggtgcaaat 120
   ggagtctagg actcagtcga ctctgcccta atgagagaag cccctgtcca agatggagaa 180
   gaagcggaga aagaaatgaa agcctctttt tgggccaagc tgtgggtgac catgggactg 240
   aggttttctt tacgttggac aagtctgtag gatggctgat cagtaaggtt gcagctttta 300
   gccaaaacag aaattcactt ctgatcaagg aagaacctag tgcgatttga atttatgcaa 360
   ttttatgacc atattcactt aggacc atg aag ctc atc aac atc tgg ctt ctt  413
                                Met Lys Leu Ile Asn Ile Trp Leu Leu
                                 1               5
   ctg ctg gtg gtt ttg ctc tgt gga aag aag cat ctg ggt gac agg ctg   461
   Leu Leu Val Val Leu Leu Cys Gly Lys Lys His Leu Gly Asp Arg Leu
    10                  15                  20                  25
   ggg aag aaa gcg ttt gaa aag gca tca tgc cct agc tgt tcc cac ctg   509
   Gly Lys Lys Ala Phe Glu Lys Ala Ser Cys Pro Ser Cys Ser His Leu
                   30                  35                  40
   act ttg aag gtg gaa ttc tcc tca act gtg gtg gaa tat gaa tat att 557
   Thr Leu Lys Val Glu Phe Ser Ser Thr Val Val Glu Tyr Glu Tyr Ile
   45 50 55
   gtg gct ttc aac gga tac ttc aca gcc aaa gct aga aac tca ttt att 605
   Val Ala Phe Asn Gly Tyr Phe Thr Ala Lys Ala Arg Asn Ser Phe Ile
   60 65 70
   tca agt gct ctg aaa agc agt gaa gta gac aac tgg aga att ata cct 653
   Ser Ser Ala Leu Lys Ser Ser Glu Val Asp Asn Trp Arg Ile Ile Pro
   75 80 85
   cgg aac aac cca tcc agt gac tac cct agt gat ttt gag gtg att cag 701
   Arg Asn Asn Pro Ser Ser Asp Tyr Pro Ser Asp Phe Glu Val Ile Gln
   90 95 100 105
   ata aaa gag aag cag aag gcc ggg ctg ctc aca ctt gaa gat cat cca 749
   Ile Lys Glu Lys Gln Lys Ala Gly Leu Leu Thr Leu Glu Asp His Pro
   110 115 120
   aac atc aag cgg gtg aca cct caa cgc aaa gtc ttt cgt tcc ttg aag 797
   Asn Ile Lys Arg Val Thr Pro Gln Arg Lys Val Phe Arg Ser Leu Lys
   125 130 135
   ttt gct gaa tct gac ccc att gtg cca tgt aat gaa act cgg tgg agc 845
   Phe Ala Glu Ser Asp Pro Ile Val Pro Cys Asn Glu Thr Arg Trp Ser
   140 145 150
   cag aag tgg cag tca tca cga ccc ctg aga aga gcc agt ctc tcc ctg 893
   Gln Lys Trp Gln Ser Ser Arg Pro Leu Arg Arg Ala Ser Leu Ser Leu
   155 160 165
   ggc tct gga ttc tgg cat gca aca gga aga cat tca agc cgg cga ttg 941
   Gly Ser Gly Phe Trp His Ala Thr Gly Arg His Ser Ser Arg Arg Leu
   170 175 180 185
   ctg aga gcc att cct cga cag gtt gcc cag aca ttg cag gca gat gtg 989
   Leu Arg Ala Ile Pro Arg Gln Val Ala Gln Thr Leu Gln Ala Asp Val
   190 195 200
   ctg tgg cag atg gga tac aca ggt gct aat gtc agg gtt gct gtt ttt 1037
   Leu Trp Gln Met Gly Tyr Thr Gly Ala Asn Val Arg Val Ala Val Phe
   205 210 215
   gat act ggg ctc agt gag aag cat cca cac ttc aag aat gtg aag gag 1085
   Asp Thr Gly Leu Ser Glu Lys His Pro His Phe Lys Asn Val Lys Glu
   220 225 230
   aga acc aac tgg acc aat gag cgg acc ctg gat gat ggg ctg ggc cat 1133
   Arg Thr Asn Trp Thr Asn Glu Arg Thr Leu Asp Asp Gly Leu Gly His
   235 240 245
   ggc aca ttt gtc gca ggt gtg att gcc agc atg agg gag tgc cag gga 1181
   Gly Thr Phe Val Ala Gly Val Ile Ala Ser Met Arg Glu Cys Gln Gly
   250 255 260 265
   ttt gcc cca gat gca gag ctg cac atc ttc cgg gtc ttt acc aac aat 1229
   Phe Ala Pro Asp Ala Glu Leu His Ile Phe Arg Val Phe Thr Asn Asn
   270 275 280
   cag gtg tct tac aca tct tgg ttt ttg gac gct ttc aac tat gcc atc    1277
   Gln Val Ser Tyr Thr Ser Trp Phe Leu Asp Ala Phe Asn Tyr Ala Ile
               285                 290                 295
   cta aag aag att gat gtt cta aac ctt agc atc ggc ggg cct gac ttc    1325
   Leu Lys Lys Ile Asp Val Leu Asn Leu Ser Ile Gly Gly Pro Asp Phe
           300                 305                 310
   atg gat cat ccc ttt gtt gac aag gtg tgg gaa tta aca gct aac aat    1373
   Met Asp His Pro Phe Val Asp Lys Val Trp Glu Leu Thr Ala Asn Asn
       315                 320                 325
   gta atc atg gtt tct gct atc ggc aat gat gga cct ctt tat ggc act    1421
   Val Ile Met Val Ser Ala Ile Gly Asn Asp Gly Pro Leu Tyr Gly Thr
   330                 335                 340                 345
   ctg aat aac cca gct gat cag atg gat gtg att gga gtg ggt ggc att    1469
   Leu Asn Asn Pro Ala Asp Gln Met Asp Val Ile Gly Val Gly Gly Ile
                   350                 355                 360
   gac ttt gaa gat aac atc gcc cgc ttt tct tcc agg gga atg act acc    1517
   Asp Phe Glu Asp Asn Ile Ala Arg Phe Ser Ser Arg Gly Met Thr Thr
               365                 370                 375
   tgg gaa cta cca gga ggc tat ggt cgc gtg aaa cct gac att gtc acc    1565
   Trp Glu Leu Pro Gly Gly Tyr Gly Arg Val Lys Pro Asp Ile Val Thr
           380                 385                 390
   tat ggt gcc gga gtg cgg ggt tcc ggt gtg aaa ggg ggc tgc cgg gca    1613
   Tyr Gly Ala Gly Val Arg Gly Ser Gly Val Lys Gly Gly Cys Arg Ala
       395                 400                 405
   ctc tca ggg acc agt gtc gct tcc cca gtg gtt gct ggg gct gtc acc    1661
   Leu Ser Gly Thr Ser Val Ala Ser Pro Val Val Ala Gly Ala Val Thr
   410                 415                 420                 425
   ttg tta gta agc aca gtg cag aag cgg gag cta gtg aat cct gcc agt    1709
   Leu Leu Val Ser Thr Val Gln Lys Arg Glu Leu Val Asn Pro Ala Ser
                   430                 435                 440
   gtg aag caa gcc ctg att gca tca gcc cgg agg ctt cct ggt gtt aac    1757
   Val Lys Gln Ala Leu Ile Ala Ser Ala Arg Arg Leu Pro Gly Val Asn
               445                 450                 455
   atg ttc gag caa ggc cat ggc aag ctg gat ctg ctg cga gcc tat cag    1805
   Met Phe Glu Gln Gly His Gly Lys Leu Asp Leu Leu Arg Ala Tyr Gln
           460                 465                 470
   atc ctc agc agc tac aaa cca cag gcg agc ttg agt cct agc tac atc    1853
   Ile Leu Ser Ser Tyr Lys Pro Gln Ala Ser Leu Ser Pro Ser Tyr Ile
       475                 480                 485
   gac ctg act gag tgt ccc tac atg tgg cct tac tgt tct cag ccc atc    1901
   Asp Leu Thr Glu Cys Pro Tyr Met Trp Pro Tyr Cys Ser Gln Pro Ile
   490                 495                 500                 505
   tac tat gga gga atg cca aca att gtt aat gtc acc atc ctc aat ggc    1949
   Tyr Tyr Gly Gly Met Pro Thr Ile Val Asn Val Thr Ile Leu Asn Gly
                   510                 515                 520
   atg gga gtc aca gga aga att gtg gat aag cct gag tgg cgg ccc tat    1997
   Met Gly Val Thr Gly Arg Ile Val Asp Lys Pro Glu Trp Arg Pro Tyr
               525                 530                 535
   tta cca cag aat gga gac aac att gaa gtg gcc ttc tcc tac tcc tca    2045
   Leu Pro Gln Asn Gly Asp Asn Ile Glu Val Ala Phe Ser Tyr Ser Ser
           540                 545                 550
   gtg tta tgg cct tgg tca ggc tac ctg gcc atc tcc att tct gtg acc    2093
   Val Leu Trp Pro Trp Ser Gly Tyr Leu Ala Ile Ser Ile Ser Val Thr
       555                 560                 565
   aag aag gca gct tcc tgg gaa ggc att gca cag ggt cac atc atg atc    2141
   Lys Lys Ala Ala Ser Trp Glu Gly Ile Ala Gln Gly His Ile Met Ile
   570                 575                 580                 585
   acg gtg gct tcc cca gca gag acg gaa gca aaa aat ggt gcc gag cat    2189
   Thr Val Ala Ser Pro Ala Glu Thr Glu Ala Lys Asn Gly Ala Glu His
                   590                 595                 600
   act tcc aca gtg aag ctt ccc att aag gtg aag atc att ccc acc cct    2237
   Thr Ser Thr Val Lys Leu Pro Ile Lys Val Lys Ile Ile Pro Thr Pro
               605                 610                 615
   cct cgg agc aag aga gtc ctc tgg gac cag tat cac aac ctc cgc tac    2285
   Pro Arg Ser Lys Arg Val Leu Trp Asp Gln Tyr His Asn Leu Arg Tyr
           620                 625                 630
   ccc cca ggc tac ttt ccc agg gac aac ttg cgg atg aag aat gat cct    2333
   Pro Pro Gly Tyr Phe Pro Arg Asp Asn Leu Arg Met Lys Asn Asp Pro
       635                 640                 645
   tta gac tgg aat ggc gac cat gtc cac acc aat ttc agg gac atg tac    2381
   Leu Asp Trp Asn Gly Asp His Val His Thr Asn Phe Arg Asp Met Tyr
   650                 655                 660                 665
   cag cac ctg cgc agc atg ggc tac ttc gtg gag gtg ctc ggt gcc cca    2429
   Gln His Leu Arg Ser Met Gly Tyr Phe Val Glu Val Leu Gly Ala Pro
                   670                 675                 680
   ttc acg tgc ttt gat gct aca cag tat ggc act ttg ctc atg gtg gat    2477
   Phe Thr Cys Phe Asp Ala Thr Gln Tyr Gly Thr Leu Leu Met Val Asp
               685                 690                 695
   agt gaa gaa gag tac ttc cca gag gag att gcc aag ctg agg agg gac    2525
   Ser Glu Glu Glu Tyr Phe Pro Glu Glu Ile Ala Lys Leu Arg Arg Asp
           700                 705                 710
   gtg gac aat ggc ctt tcc ctc gtc atc ttc agt gac tgg tac aac act    2573
   Val Asp Asn Gly Leu Ser Leu Val Ile Phe Ser Asp Trp Tyr Asn Thr
       715                 720                 725
   tct gtt atg aga aaa gtg aag ttt tac gat gaa aac aca agg cag tgg    2621
   Ser Val Met Arg Lys Val Lys Phe Tyr Asp Glu Asn Thr Arg Gln Trp
   730                 735                 740                 745
   tgg atg cca gat act gga gga gcc aac atc cca gct ctg aac gag ctg     2669
   Trp Met Pro Asp Thr Gly Gly Ala Asn Ile Pro Ala Leu Asn Glu Leu
                   750                 755                 760
   ctg tct gtg tgg aac atg ggg ttc agc gat ggc ctt tat gaa ggg gag     2717
   Leu Ser Val Trp Asn Met Gly Phe Ser Asp Gly Leu Tyr Glu Gly Glu
               765                 770                 775
   ttt gcc ctg gcg aat cat gac atg tat tat gca tcg gga tgc agc atc     2765
   Phe Ala Leu Ala Asn His Asp Met Tyr Tyr Ala Ser Gly Cys Ser Ile
           780                 785                 790
   gcc aag ttt cca gaa gat ggt gtt gtg atc aca cag act ttc aag gac     2813
   Ala Lys Phe Pro Glu Asp Gly Val Val Ile Thr Gln Thr Phe Lys Asp
       795                 800                 805
   caa gga ttg gag gtc tta aaa caa gag aca gca gtt gtt gaa aat gtt     2861
   Gln Gly Leu Glu Val Leu Lys Gln Glu Thr Ala Val Val Glu Asn Val
   810                 815                 820                 825
   ccc att ttg ggg ctt tat cag att cca gct gaa ggt ggg ggc cgg atc     2909
   Pro Ile Leu Gly Leu Tyr Gln Ile Pro Ala Glu Gly Gly Gly Arg Ile
                   830                 835                 840
   gtg ttg tat gga gat tcc aat tgc ttg gat gac agt cac aga cag aag     2957
   Val Leu Tyr Gly Asp Ser Asn Cys Leu Asp Asp Ser His Arg Gln Lys
               845                 850                 855
   gat tgc ttt tgg ctt ctg gat gca ctc ctt cag tac aca tca tat ggc     3005
   Asp Cys Phe Trp Leu Leu Asp Ala Leu Leu Gln Tyr Thr Ser Tyr Gly
           860                 865                 870
   gtg aac cct ccc agc ctc agc cat tca ggg aac cgg cag cgc cca ccc     3053
   Val Asn Pro Pro Ser Leu Ser His Ser Gly Asn Arg Gln Arg Pro Pro
       875                 880                 885
   agt gga gct ggc ttg gcc cct cct gaa agg atg gaa gga aac cac ctt     3101
   Ser Gly Ala Gly Leu Ala Pro Pro Glu Arg Met Glu Gly Asn His Leu
   890                 895                 900                 905
   cat cga tac tcc aag gtt ctt gag gcc cat ctg gga gac cca aaa cct     3149
   His Arg Tyr Ser Lys Val Leu Glu Ala His Leu Gly Asp Pro Lys Pro
                   910                 915                 920
   cgg cct ctt cca gcc tgt cca cac ttg tca tgg gcc aag cca cag cct     3197
   Arg Pro Leu Pro Ala Cys Pro His Leu Ser Trp Ala Lys Pro Gln Pro
               925                 930                 935
   ttg aat gag act gcg ccc agt aat ctt tgg aaa cat cag aag ctg ctc     3245
   Leu Asn Glu Thr Ala Pro Ser Asn Leu Trp Lys His Gln Lys Leu Leu
           940                 945                 950
   tcc att gac ctg gac aaa gta gtg tta ccc aac ttt cga tcg aat cgc     3293
   Ser Ile Asp Leu Asp Lys Val Val Leu Pro Asn Phe Arg Ser Asn Arg
       955                 960                 965
   cct caa gtg aga cct ttg tcc cct gga gaa agt ggt gcc tgg gac att   3341
   Pro Gln Val Arg Pro Leu Ser Pro Gly Glu Ser Gly Ala Trp Asp Ile
   970                 975                 980                 985
   cct gga ggg atc atg cct ggc cgc tac aac caa gag gtg ggc cag acc   3389
   Pro Gly Gly Ile Met Pro Gly Arg Tyr Asn Gln Glu Val Gly Gln Thr
                   990                 995                 1000
   atc cct gtc ttt gcc ttc ctc gga gcc atg gtg gcc ctg gcc ttc ttt   3437
   Ile Pro Val Phe Ala Phe Leu Gly Ala Met Val Ala Leu Ala Phe Phe
               1005                1010                1015
   gtg gta cag atc agc aag gcc aaa agc cgg ccg aag cgg agg agg ccc   3485
   Val Val Gln Ile Ser Lys Ala Lys Ser Arg Pro Lys Arg Arg Arg Pro
           1020                1025                1030
   agg gca aag cgt cca cag ctt aca cag cag acc cac cca cca agg acc   3533
   Arg Ala Lys Arg Pro Gln Leu Thr Gln Gln Thr His Pro Pro Arg Thr
       1035                1040                1045
   ccg tca gtg tga tcatcacagt ggccagccac agaagccaac aagccttgga       3585
   Pro Ser Val
   1050
   ccactctgat ggccacacag ggcatcagaa gagcatcctg ggaggtgcct atttccaagg 3645
   gaccccatct ccagcttgtg gctgggttag tgtgttctcc ccaggcatct ctgagttaca 3705
   tcctgaagta cctcactgtg ctgggctctt gacaggaggt gctcagtagc tcagcctcca 3765
   gtggtgtcag caggcccagt gacagtgcac caaagacaca gagcctggaa gggctgtcgg 3825
   gacacacttt ctacataaag cttacaatcc tgaccaagcg aagaaatgct tgttacaggc 3885
   tattttctat atttattgtg gggagagtca ctttaaagac ttgtactgtt tggaagcaaa 3945
   gctgttgtgt ttgtcagttg agtgcagttt tctgcagtga catcataagg agtcagatcc 4005
   catgaccttt ttgatgagag gacagactga actgaagggc atgtgcacag atctgggaaa 4065
   tgcaagcctt cgctttattt ttataagtat caactgccat catgttttgt aatttggggt 4125
   cttgatttca ccattgttgg tgaaagaaat tttcaataaa tatgcataac cttaaaaaaa 4185
   aaaaaaaaaa aaa                                                    4198
<210> 6
   <211> 1052
   <212> PRT
   <213> Cricetulus griseus
<400> 6
   Met Lys Leu Ile Asn Ile Trp Leu Leu Leu Leu Val Val Leu Leu Cys
    1               5                  10                  15
   Gly Lys Lys His Leu Gly Asp Arg Leu Gly Lys Lys Ala Phe Glu Lys
               20                  25                  30
   Ala Ser Cys Pro Ser Cys Ser His Leu Thr Leu Lys Val Glu Phe Ser
           35                  40                  45
   Ser Thr Val Val Glu Tyr Glu Tyr Ile Val Ala Phe Asn Gly Tyr Phe
        50                  55                  60
   Thr Ala Lys Ala Arg Asn Ser Phe Ile Ser Ser Ala Leu Lys Ser Ser
    65                  70                  75                  80
   Glu Val Asp Asn Trp Arg Ile Ile Pro Arg Asn Asn Pro Ser Ser Asp
                    85                  90                  95
   Tyr Pro Ser Asp Phe Glu Val Ile Gln Ile Lys Glu Lys Gln Lys Ala
               100                 105                 110
   Gly Leu Leu Thr Leu Glu Asp His Pro Asn Ile Lys Arg Val Thr Pro
           115                 120                 125
   Gln Arg Lys Val Phe Arg Ser Leu Lys Phe Ala Glu Ser Asp Pro Ile
       130                 135                 140
   Val Pro Cys Asn Glu Thr Arg Trp Ser Gln Lys Trp Gln Ser Ser Arg
   145                 150                 155                 160
   Pro Leu Arg Arg Ala Ser Leu Ser Leu Gly Ser Gly Phe Trp His Ala
                   165                 170                 175
   Thr Gly Arg His Ser Ser Arg Arg Leu Leu Arg Ala Ile Pro Arg Gln
               180                 185                 190
   Val Ala Gln Thr Leu Gln Ala Asp Val Leu Trp Gln Met Gly Tyr Thr
           195                 200                 205
   Gly Ala Asn Val Arg Val Ala Val Phe Asp Thr Gly Leu Ser Glu Lys
       210                 215                 220
   His Pro His Phe Lys Asn Val Lys Glu Arg Thr Asn Trp Thr Asn Glu
   225                 230                 235                 240
   Arg Thr Leu Asp Asp Gly Leu Gly His Gly Thr Phe Val Ala Gly Val
                   245                 250                 255
   Ile Ala Ser Met Arg Glu Cys Gln Gly Phe Ala Pro Asp Ala Glu Leu
               260                 265                 270
   His Ile Phe Arg Val Phe Thr Asn Asn Gln Val Ser Tyr Thr Ser Trp
           275                 280                 285
   Phe Leu Asp Ala Phe Asn Tyr Ala Ile Leu Lys Lys Ile Asp Val Leu
       290                 295                 300
   Asn Leu Ser Ile Gly Gly Pro Asp Phe Met Asp His Pro Phe Val Asp
   305                 310                 315                 320
   Lys Val Trp Glu Leu Thr Ala Asn Asn Val Ile Met Val Ser Ala Ile
                   325                 330                 335
   Gly Asn Asp Gly Pro Leu Tyr Gly Thr Leu Asn Asn Pro Ala Asp Gln
               340                 345                 350
   Met Asp Val Ile Gly Val Gly Gly Ile Asp Phe Glu Asp Asn Ile Ala
           355                 360                 365
   Arg Phe Ser Ser Arg Gly Met Thr Thr Trp Glu Leu Pro Gly Gly Tyr
       370                 375                 380
   Gly Arg Val Lys Pro Asp Ile Val Thr Tyr Gly Ala Gly Val Arg Gly
   385                 390                 395                 400
   Ser Gly Val Lys Gly Gly Cys Arg Ala Leu Ser Gly Thr Ser Val Ala
                   405                 410                 415
   Ser Pro Val Val Ala Gly Ala Val Thr Leu Leu Val Ser Thr Val Gln
               420                 425                 430
   Lys Arg Glu Leu Val Asn Pro Ala Ser Val Lys Gln Ala Leu Ile Ala
           435                 440                 445
   Ser Ala Arg Arg Leu Pro Gly Val Asn Met Phe Glu Gln Gly His Gly
       450                 455                 460
   Lys Leu Asp Leu Leu Arg Ala Tyr Gln Ile Leu Ser Ser Tyr Lys Pro
   465                 470                 475                 480
   Gln Ala Ser Leu Ser Pro Ser Tyr Ile Asp Leu Thr Glu Cys Pro Tyr
                   485                 490                 495
   Met Trp Pro Tyr Cys Ser Gln Pro Ile Tyr Tyr Gly Gly Met Pro Thr
               500                 505                 510
   Ile Val Asn Val Thr Ile Leu Asn Gly Met Gly Val Thr Gly Arg Ile
   515 520 525
   Val Asp Lys Pro Glu Trp Arg Pro Tyr Leu Pro Gln Asn Gly Asp Asn
   530 535 540
   Ile Glu Val Ala Phe Ser Tyr Ser Ser Val Leu Trp Pro Trp Ser Gly
   545 550 555 560
   Tyr Leu Ala Ile Ser Ile Ser Val Thr Lys Lys Ala Ala Ser Trp Glu
   565 570 575
   Gly Ile Ala Gln Gly His Ile Met Ile Thr Val Ala Ser Pro Ala Glu
   580 585 590
   Thr Glu Ala Lys Asn Gly Ala Glu His Thr Ser Thr Val Lys Leu Pro
   595 600 605
   Ile Lys Val Lys Ile Ile Pro Thr Pro Pro Arg Ser Lys Arg Val Leu
   610 615 620
   Trp Asp Gln Tyr His Asn Leu Arg Tyr Pro Pro Gly Tyr Phe Pro Arg
   625 630 635 640
   Asp Asn Leu Arg Met Lys Asn Asp Pro Leu Asp Trp Asn Gly Asp His
   645 650 655
   Val His Thr Asn Phe Arg Asp Met Tyr Gln His Leu Arg Ser Met Gly
   660 665 670
   Tyr Phe Val Glu Val Leu Gly Ala Pro Phe Thr Cys Phe Asp Ala Thr
   675 680 685
   Gln Tyr Gly Thr Leu Leu Met Val Asp Ser Glu Glu Glu Tyr Phe Pro
   690 695 700
   Glu Glu Ile Ala Lys Leu Arg Arg Asp Val Asp Asn Gly Leu Ser Leu
   705 710 715 720
   Val Ile Phe Ser Asp Trp Tyr Asn Thr Ser Val Met Arg Lys Val Lys
   725 730 735
   Phe Tyr Asp Glu Asn Thr Arg Gln Trp Trp Met Pro Asp Thr Gly Gly
   740 745 750
   Ala Asn Ile Pro Ala Leu Asn Glu Leu Leu Ser Val Trp Asn Met Gly
   755 760 765
   Phe Ser Asp Gly Leu Tyr Glu Gly Glu Phe Ala Leu Ala Asn His Asp
   770 775 780
   Met Tyr Tyr Ala Ser Gly Cys Ser Ile Ala Lys Phe Pro Glu Asp Gly
   785 790 795 800
   Val Val Ile Thr Gln Thr Phe Lys Asp Gln Gly Leu Glu Val Leu Lys
   805 810 815
   Gln Glu Thr Ala Val Val Glu Asn Val Pro Ile Leu Gly Leu Tyr Gln
   820 825 830
   Ile Pro Ala Glu Gly Gly Gly Arg Ile Val Leu Tyr Gly Asp Ser Asn
   835 840 845
   Cys Leu Asp Asp Ser His Arg Gln Lys Asp Cys Phe Trp Leu Leu Asp
   850 855 860
   Ala Leu Leu Gln Tyr Thr Ser Tyr Gly Val Asn Pro Pro Ser Leu Ser
   865 870 875 880
   His Ser Gly Asn Arg Gln Arg Pro Pro Ser Gly Ala Gly Leu Ala Pro
   885 890 895
   Pro Glu Arg Met Glu Gly Asn His Leu His Arg Tyr Ser Lys Val Leu
   900 905 910
   Glu Ala His Leu Gly Asp Pro Lys Pro Arg Pro Leu Pro Ala Cys Pro
   915 920 925
   His Leu Ser Trp Ala Lys Pro Gln Pro Leu Asn Glu Thr Ala Pro Ser
   930 935 940
   Asn Leu Trp Lys His Gln Lys Leu Leu Ser Ile Asp Leu Asp Lys Val
   945 950 955 960
   Val Leu Pro Asn Phe Arg Ser Asn Arg Pro Gln Val Arg Pro Leu Ser
   965 970 975
   Pro Gly Glu Ser Gly Ala Trp Asp Ile Pro Gly Gly Ile Met Pro Gly
               980                 985                 990
   Arg Tyr Asn Gln Glu Val Gly Gln Thr Ile Pro Val Phe Ala Phe Leu
           995                 1000                1005
   Gly Ala Met Val Ala Leu Ala Phe Phe Val Val Gln Ile Ser Lys Ala
      1010                 1015                1020
   Lys Ser Arg Pro Lys Arg Arg Arg Pro Arg Ala Lys Arg Pro Gln Leu
   1025                1030                1035                1040
   Thr Gln Gln Thr His Pro Pro Arg Thr Pro Ser Val
                   1045                1050

## Description

### TECHNICAL FIELD

The present invention relates an isolated human Site-1 Protease promoter region. The invention also relates to screening methods for agents decreasing the expression of Site-1 protease and thereby being potentially useful for the treatment of medical conditions related to obesity and / or diabetes.

### BACKGROUND ART

### Sterol Regulatory Element-Binding Proteins (SREBPs)

The integrity of cell membranes is maintained by a balance between the amount of cholesterol and the amounts of unsaturated and saturated fatty acids in phospholipids. This balance is partly maintained by membrane-bound transcription factors called Sterol Regulatory Element-Binding Proteins (SREBPs; for reviews, see Brown & Goldstein (1997) Cell 89, 331-340; Brown & Goldstein (1999) Proc. Natl. Acad. Sci. U.S.A. 96, 11041-11048) that activate genes encoding enzymes of cholesterol and fatty acid biosynthesis. To enhance transcription, the active NH₂-terminal domains of SREBPs are released from endoplasmic reticulum membranes by two sequential cleavages. The first is catalyzed by Site-1 protease (S1P), a membrane-bound subtilisin-related serine protease that cleaves the hydrophilic loop of SREBP that projects into the endoplasmic reticulum lumen. The second cleavage, at Site-2, requires the action of S2P, a hydrophobic protein that appears to be a zinc metalloprotease. These regulated proteolytic cleavage reactions are ultimately responsible for controlling the level of cholesterol in membranes, cells, and blood.

Three isoforms of SREBPs have been identified. SREBP-1a and SREBP-1c are encoded by a single gene and differ in their N-terminal acid transcription activation domains.
The N-terminus of SREBP-1a is longer and includes additional acidic amino acids, consistent with the observation that it is a more powerful transcription factor (Pai et al. (1998) Proc. Natl. Acad. Sci. U.S.A. 40, 26138-26148). SREBP-2 is produced by a different gene and contains a long activation domain resembling that of SREBP-1a. Recent evidence suggests that the main function of SREBP-2 is to regulate cholesterol synthesis whilst that of SREBP-1 is to regulate fatty acid synthesis (Pai et al., *supra*).

Inhibition of SREBP transcription factor function will lead to reduced cellular synthesis of free fatty acids and cholesterol, the clinical benefits of which are expected to include increased cellular insulin sensitivity and reduced coronary artery disease (CAD). Furthermore, SREBP-1 represents a cellular mechanism for increasing both fat cell size and number (Kim et al. (1998) J. Clin. Invest. 101, 1-9). Since most obesity generally involves an increase in both cell size and cell number, inhibition of SREBP-1 might be expected to have a positive effect on obesity. The hypolipidemic effects of dietary polyunsaturated fatty acids are believed to derive from a direct inhibitory effect on SREBP-1 expression (Xu et al. (1999) J. Biol. Chem. 274, 23577-23583).

There is data indicating independent regulation of SREBP-1 and SREBP-2 in hamster liver, suggesting the possibility for specific targeting of SREBP-1 or -2 (Sheng et al. (1995) Proc. Natl. Acad. Sci. U.S.A. 92, 935-938).

Transgenic mice over-expressing a dominant-positive form of SREBP-2 in the liver and adipose tissue showed greatly increased levels of mRNAs encoding multiple enzymes of cholesterol synthesis. Enzymes involved in fatty acid synthesis were also increased, however, to a lesser extent (Horton et al. (1998) J. Clin. Invest. 101, 2331-2339). Transgenic mice over-expressing a constitutively active SREBP-1a in the liver and adipose tissue showed greatly increased mRNA levels for enzymes involved in fatty acid and cholesterol (Shimano et al. (1996) J. Clin. Invest. 98, 1575-1584). Their livers were enlarged about 4-fold due to a massive accumulation of free fatty acids and cholesterol. Over-expression of a corresponding version of SREBP-1c in adipocytes of transgenic mice yielded insulin resistance and diabetes (Shimomura et al. (1999) Genes Dev. 12, 3182-3194). In cell culture such overexpression was previously shown to promote adipocyte differentiation. It has further been shown that ovemutrition increases SREBP-1c expression in liver and islets of obese fa/fa Zucker diabetic fatty rats (Kakuma, T. et al. (2000) Proc. Natl. Acad. Sci. U.S.A. 97: 8536-8541).

### S-1 Protease

As discussed above, SREBPs are activated by proteolysis, which releases the active transcription factor. The luminal subtilisin-like protease Site-1 Protease (S1P) is responsible for the first of the two proteolytic steps. Cleavage by S1P enables further cleavage by a Site-2 protease. S1P is the target for feedback inhibition by cholesterol.

S1P from hamster has been cloned (Sakai et al. (1998) Molecular Cell 2, 505-514). (GenBank accession no. AF078105; SEQ ID NOS: 5 and 6). The corresponding sequence of the (then unidentified) human gene was disclosed by Nagase et al. (1995) DNA Research 2, 37-43 (GenBank Accession no. D420453; SEQ ID NOS: 3 and 4)

SREBP and S1P are co-localized with a third protein: SREBP Cleavage-Activating Protein (SCAP), which is required for Site-1 cleavage *in vivo.* SCAP contains a site for sterol regulation, conserved in a small number of proteins, e.g. HMG-CoA reductase.

Only one S1P has been identified among the human expressed sequence tags (ESTs). Thus, S1P may be the only member of a subfamily among the subtilisin-like proteases.

Consequently, SREBPs are important regulators of fat and sugar metabolism in mammals and direct or indirect down-regulation of SREBPs may be of therapeutic value in type II diabetes; obesity, hypercholesterolemia, and other cardiovascular diseases or dyslipidemias.

Site-1 Protease represents a molecular target for therapeutic intervention which is expected to interfere with the SREBP pathway. Two principally distinct concepts for inhibition of the site-1-protease activity may be postulated; (i) by inactivation of the proteolytic activity (classical inhibitors) or (ii) by modulation of the site-1-protease gene expression level. In order to modulate the expression of the site-1-protease gene, there is a need for identification of regulatory regions responsible for the regulation of Site-1 protease promoter. Such regulatory regions in the promoter could be used for the identification of agents that inhibit expression of Site-1 protease, and thereby for the inhibition of the SREBP pathway.

### DISCLOSURE OF THE INVENTION

The 5'-flanking region (promoter region) of the human Site-1 Protease (S1P) gene has been cloned and sequenced. This promoter region is useful in biological assays for the identification of compounds that inhibit the transcription of the Site-1 Protease. Inhibition of the SREBP pathway is expected to have therapeutic value in type II diabetes; obesity, hypercholesterolemia, and other cardiovascular diseases or dyslipidemias.

Consequently, in a first aspect this invention provides an isolated human site-1 protease promoter region comprising a sequence selected from:
(a) the nucleotide sequence set forth as SEQ ID NO: 2, or a fragment thereof exhibiting site-1 protease promoter activity;
(b) the complementary strand of (a); and
(c) nucleotide sequences capable of hybridizing, under stringent hybridization conditions, to a nucleotide sequence as defined in (a) or (b).

The term "promoter region" refers to a region of DNA that functions to control the transcription of one or more genes, and is structurally identified by the presence of a binding site for DNA-dependent RNA polymerase and of other DNA sequences on the same molecule which interact to regulate promoter function.

The nucleic acid molecules according to the present invention includes cDNA, chemically synthesized DNA, DNA isolated by PCR, genomic DNA, and combinations thereof. Genomic DNA may be obtained by screening a genomic library with the cDNA described herein, using methods that are well known in the art.

In a preferred form of the invention, the said nucleic acid molecule has a nucleotide sequence identical with SEQ ID NO: 2 of the Sequence Listing. However, the nucleic acid molecule according to the invention is not to be limited strictly to the sequence shown as SEQ ID NO: 2. Rather the invention encompasses nucleic acid molecules carrying modifications like substitutions, small deletions, insertions or inversions, which nevertheless have S1P promoter activity. Included in the invention are consequently nucleic acid molecules, the nucleotide sequence of which is at least 90% homologous, preferably at least 95% homologous, with the nucleotide sequence shown as SEQ ID NO: 2 in the Sequence Listing.

The term "stringent hybridization conditions" is known in the art from standard protocols (e.g. Ausubel et al., *supra*) and could be understood as e.g. hybridization to filter-bound DNA in 0.5 M NaHPO₄, 7% sodium dodecyl sulfate (SDS), 1 mM EDTA at +65°C, and washing in 0.1xSSC / 0.1% SDS at +68°C.

The said "fragment" (partial sequence) exhibiting site-1 protease promoter activity can be identified by the skilled person by computer-assisted sequence analysis, e.g. prediction of transcription factor binding sites.

The invention further provides a recombinant construct comprising the human site-1 protease promoter region as defined above. Preferably, the said construct comprises the S1P promoter region operably linked to a gene encoding a detectable product, in particular the human site-1 protease gene (SEQ ID NO: 3).

The term "linked" indicates that a nucleotide sequence encoding a gene product and an S1P promoter, or an active fragment thereof, are located within a continuous nucleic acid sequence. The term "operably linked" means that a nucleotide sequence, which can encode a gene product, is linked to the S1P promoter such that the S1P promoter regulates expression of the gene product under appropriate conditions. Two nucleotide sequences that are operably linked contain elements essential for transcription, including, for example, a TATA box.

The recombinant construct according to the invention could further comprise a reporter gene. As used herein, the term "reporter gene" means a gene encoding a gene product that can be identified using simple, inexpensive methods or reagents and that can be operably linked to a S1P promoter or an active fragment thereof. Reporter genes such as, for example, a luciferase, β-galactosidase, alkaline phosphatase, or green fluorescent protein reporter gene, can be used to determine transcriptional activity in screening assays according to the invention (see, for example, Goeddel (ed.), Methods Enzymol., Vol. 185, San Diego: Academic Press, Inc. (*1990*); see also Sambrook, *supra*).

In another aspect the invention provides a vector comprising the recombinant construct as defined above. The term "vector" refers to any carrier of exogenous DNA that is useful for transferring the DNA to a host cell for replication and/or appropriate expression of the exogenous DNA by the host cell. A host cell stably transformed with the recombinant construct is an additional aspect of the invention. Such a host cell can be a prokaryotic cell, a unicellular eukaryotic cell, or a cell derived from a multicellular organism. The methods employed to effect introduction of the vector into the host cell are standard methods well known to a person familiar with recombinant DNA methods. The term "transformed" or "transfected" refers to the process by which exogenous DNA is transferred into an appropriate host cell.

In a further important aspect, this invention is useful in screening for pharmacological agents that modulate S1P levels by affecting the transcription of the S1P gene. As used herein, the term "agent" means a biological or chemical compound such as a simple or complex organic molecule, a peptide, a protein or an oligonucleotide. Consequently, this invention includes a method for identifying an agent capable of modulating the S1P promoter, comprising providing a cell comprising the S1P promoter; contacting said cell with a candidate agent; and monitoring said cell for an effect that is not present in the absence of said candidate agent.

A preferred form of the invention include a method for identification of an agent capable of decreasing or inhibiting site-1 protease promoter activity, said method comprising the steps (i) contacting a candidate agent with the human site-1 protease promoter; and (ii) determining whether said candidate agent decreases expression of the site-1 protease gene, such decrease being indicative for an agent capable of decreasing or inhibiting site-1 protease promoter activity.

For screening purposes, appropriate host cells can be transformed with a vector having a reporter gene under the control of the human S1P promoter according to this invention. The expression of the reporter gene can be measured in the presence or absence of an agent with known activity (i.e. a standard agent) or putative activity (i.e. a "test agent" or "candidate agent"). A change in the level of expression of the reporter gene in the presence of the test agent is compared with that effected by the standard agent. In this way, active agents are identified and their relative potency in this assay determined.

It will be understood that agents acting on the human S1P promoter can be identified by, as an additional step, analyzing direct binding interactions between the candidate agent and the human S1P promoter. Interactions with large molecules may be studied using techniques such as gel shift analysis, footprinting or NMR (see Latchman, D.S. (Ed.) (1995) Methods for studying transcription factors. In: Eukaryotic transcription factors. Academic Press, London, pp. 17-44). Small molecule compounds which appear to bind reversibly to double stranded DNA without intercalation between DNA base pairs have been defined. Methods are described by which this non-intercalative binding can be characterized using ultraviolet spectrometry, fluorimetry with ethidium as a probe, viscometry and other hydrodynamic techniques, circular dichroism and nuclear magnetic resonance spectrometry (See Baguley, B.C. (1982) Nonintercalative DNA-binding antitumour compounds. Mol Cell Biochem 43: 167-181; Gmeiner, W.H. (1998) NMR spectroscopy as a tool to investigate the structural basis of anticancer drugs. Curr Med Chem 5(2): 115-135; Wemmer, D.E. & Williams, P.G. (1994) Use of nuclear magnetic resonance in probing ligand-macromolecule interactions. Methods Enzymol. 239:739-767)

A potentially useful method for identification of agents acting on the human S1P promoter is described in Swedish patent application No. 0101218-6, filed on 5 April 2001. Such a method comprises the steps
(a) predicting the structure of an RNA-fragment;
(b) choosing a suitable predicted RNA-fragment of step (a), which RNA-fragment comprises at least one individual stem;
(c) synthesizing the DNA-fragment corresponding to the RNA-fragment of step (b);
(d) inserting the DNA-fragment of step (c) in the upstream proximity of a reporter assay gene, which reporter assay gene produces a signal upon translation, thereby forming a reporter construct;
(e) performing a reporter gene assay, which assay monitors the interaction between a molecule to be tested for RNA-binding and the RNA-fragment of the reporter construct.

As mentioned above, it is expected that agents capable of decreasing or inhibiting site-1 protease promoter activity have potential therapeutic value in particular in obesity, and in type II diabetes; hypercholesterolemia, atherosclerosis and other cardiovascular diseases or dyslipidemias. Consequently, the invention also comprises a method for identification of an agent useful for the treatment of medical conditions related to obesity according to claims 12.

Throughout this description the terms "standard protocols" and "standard procedures", when used in the context of molecular biology techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

### EXAMPLES

### EXAMPLE 1: Cloning of S1P promoter fragment

For cloning of the 5'-flanking region of the S1-protease gene, a genomic walking strategy was used, principally as described by Siebert et al. (1995) Nucleic Acids Res. 23,1087-1088; and Siebert et al. (1995) CLONTECHniques X, 1-3. Two primers, designated FOMA 345 and FOMA 346, were selected in the 5'-region of the cDNA: All other reagents used were obtained with a "Genome Walker Kit" (Clontech, Palo Alto, CA). The principle of this procedure is to perform two subsequent PCR reactions using adaptor-ligated genomic DNA as template. In the first PCR reaction the "outer" primers are used, i.e. FOMA 345 and AP1 (adaptor primer 1). The protocol for this reaction was:
(+95° for 25 sec; +72° for 4 min) x 7 cycles (+95° for 25 sec; +67° for 4 min) x 35 cycles (+67° for 4 min) x 1 cycle

In the second PCR reaction, the "inner" primers were used (FOMA 346 and AP2). The reaction mix from the first PCR was diluted 50 times and 1 µl of this cocktail was used as template in the second reaction. The protocol of the second reaction was:
(+95° for 25 sec; +72° for 4 min) x 5 cycles
(+95° for 25 sec; +67° for 4 min) x 25 cycles
(+67° for 4 min) x 1 cycle

The reaction mixes were prepared in accordance with the instructions of the kit manufacturer. After the second PCR, the product was analyzed by electrophoresis in 2% agarose gel. A product, approximately 1 kb long, was observed in one of the adaptor-ligated genomic DNA-libraries (HDL2). This product was cloned into the TOPO vector PCR2.1 (Invitrogen, Carlsbad, CA) by standard cloning procedures and thereafter sequenced. A 980 bp sequence was obtained (SEQ ID NO: 1).

### EXAMPLE 2: Assembly of S1P promoter sequence

The Celera database (Release 1.13) was searched using the 980 bp sequence obtained in Example 1 as query sequence. The BLAST algorithm (Altschul et al. (1997) Nucleic Acids Res. 25:3389-3402) was used for determining sequence identity. Software for performing BLAST analyses is publicly available through the National Center for Biotechnology Information (http://www.ncbi.nlm.nih.gov/). Six fragments (GA_16330554; GA_25791426; GA_23194195; GA_28969362; GA_18902492; GA_24454650) that overlapped with the genomic sequence were retrieved. These six fragments were used to search the Celera database (Release 1.13) again, the overlapping sequences were extended with another 7 fragments (GA_24421404, GA_21984802, GA_28735370, GA_21045430, GA_9491232, GA_13453697, GA_25224137). All 13 fragments together with the 980 bp sequence obtained in Example 1 were finally assembled to a 2469 bp contig (SEQ ID NO: 2) using the Cap2 program (Huang (1996) Genomics 33, 21-31).

### EXAMPLE 3: Reporter gene assay to identify modulating compounds

Reporter gene assays are well known as tools to signal transcriptional activity in cells. (For a review of chemiluminescent and bioluminescent reporter gene assays, see Bronstein et al. (1994) Analytical Biochemistry 219, 169-181.) For instance, the photoprotein luciferase provides a useful tool for assaying for modulators of S1P promoter activity. Cells (e.g. CHO cells or COS 7 cells) are transiently co-transfected with both a Site-1 protease expression construct and a reporter construct which includes a gene for the luciferase protein downstream from a transcription factor binding site. Luciferase activity may be quantitatively measured using e.g. luciferase assay reagents that are commercially available from Promega (Madison, WI). Differences in luminescence in the presence versus the absence of a candidate modulator compound are indicative of modulatory activity.

A luciferase reporter plasmid is prepared by cloning a 980 bp sequence (SEQ ID NO: 1) corresponding to a part of the site-1-protease promoter into the pGL2 vector, in which the luciferase reporter gene is driven by the activity of the inserted promoter. The construct is thereafter transfected into the mouse pre-adipocyte cell line 3T3-L1 (ATCC No. CCL92.1), the human embryonic kidney cell line 293 (ATCC No. CRL-1573), and the human hepatoma cell line HepG2 (ATCC No. HB-8065). Altered promoter activity after stimulation with a number of substances, including insulin, glitazones and sterols, are measured as changes in the readout of luciferase.

## Claims

1. An isolated human site-1 protease promoter region comprising a sequence selected from:
(a) the nucleotide sequence set forth as SEQ ID NO: 2, or a fragment thereof exhibiting site-1 protease promoter activity;
(b) the complementary strand of (a); and
(c) nucleotide sequences capable of hybridizing, under stringent hybridization conditions, to a nucleotide sequence as defined in (a) or (b).

2. A recombinant construct comprising the human site-1 protease promoter region according to claim 1.

3. The recombinant construct according to claim 2 wherein the human site-1 protease promoter region is operably linked to a gene encoding a detectable product.

4. The recombinant construct according to claim 3 wherein said gene encoding a detectable product is a human site-1 protease gene.

5. The recombinant construct according to claim 2 further comprising a reporter gene.

6. A vector comprising the recombinant construct according to any one of claims 2 to 5.

7. A host cell stably transformed with the recombinant construct according to any one of claims 2 to 5.

8. A host cell stably transformed with the vector according to claim 6.

9. A method for identification of an agent capable of decreasing or inhibiting site-1 protease promoter activity, said method comprising the steps
(i) contacting a candidate agent with the human site-1 protease promoter region as defined in claim 1; and
(ii) determining whether said candidate agent decreases expression of the site-1 protease gene, such decrease being indicative for an agent capable of decreasing or inhibiting site-1 protease promoter activity.

10. A method for identification of an agent useful for the treatment of a medical condition related to obesity, said method comprising the steps
(i) contacting a candidate agent with the human site-1 protease promoter region as defined in claim 1; and
(ii) determining whether said candidate agent decreases expression of the site-1 protease gene, such decrease being indicative for an agent useful for the treatment of a medical condition related to obesity.

11. A method for identification of an agent capable of decreasing or inhibiting site-1 protease promoter activity, said method comprising assaying reporter gene expression in a cell according to claim 7 or 8 in the presence and absence of a candidate agent, wherein a decrease or inhibition of test level of expression as compared to control level of expression is indicative of an agent capable of decreasing or inhibiting site-1 protease promoter activity.

12. A method for identification of an agent useful for the treatment of medical conditions related to obesity, said method comprising assaying reporter gene expression in a cell according to claim 7 or 8 in the presence and absence of a candidate agent, wherein a decrease or inhibition of test level of expression as compared to control level of expression is indicative of an agent useful for the treatment of medical conditions related to obesity.

## Patentansprüche

1. Isolierte menschliche Site-1-Protease-Promotor-Region, umfassend eine Sequenz, die gewählt ist aus:
(a) der in SEQ. ID. Nr.: 2 dargestellten Nukleotidsequenz, oder einem Fragment davon, welches Site-1-Protease-Promotor-Aktivität besitzt;
(b) dem komplementären Strang von (a); und
(c) Nukleotidsequenzen, die unter stringenten Hybridisierungsbedingungen in der Lage sind, an einer Nukleotidsequenz, wie sie in (a) oder (b) definiert ist, zu hybridisieren.

2. Rekombinantes Konstrukt, umfassend die menschliche Site-1-Protease-Promotor-Region gemäß Anspruch 1.

3. Rekombinantes Konstrukt gemäß Anspruch 2, wobei die menschliche Site-1-Protease-Promotor-Region funktionsfähig an ein Gen geknüpft ist, welches ein detektierbares Produkt kodiert.

4. Rekombinantes Konstrukt gemäß Anspruch 3, wobei das ein detektierbares Produkt kodierende Gen ein menschliches Site-1-Protease-Gen ist.

5. Rekombinantes Konstrukt gemäß Anspruch 2, ferner umfassend ein Reporter-Gen.

6. Vektor, umfassend das rekombinante Konstrukt gemäß mindestens einem der Ansprüche 2 bis 5.

7. Wirtszelle, welche stabil mit dem rekombinanten Konstrukt gemäß mindestens einem der Ansprüche 2 bis 5 transformiert wurde.

8. Wirtszelle, welche stabil mit dem Vektor gemäß Anspruch 6 transformiert wurde

9. Verfahren zum Identifizieren eines Mittels, das zur Verringerung oder Inhibierung der Site-1-Protease-Promotor-Aktivität in der Lage ist, wobei das Verfahren die folgenden Schritte umfasst:
(i) Kontaktieren eines Kandidatenmittels mit der menschlichen Site-1-Protease-Promotor-Region, wie in Anspruch 1 definiert; und
(ii) Bestimmen, ob das Kandidatenmittel die Expression des Site-1-Protease-Gens verringert, wobei eine solche Verringerung auf ein Mittel hinweist, das zur Verringerung oder Inhibierung der Site-1-Protease-Promotor-Aktivität in der Lage ist.

10. Verfahren zum Identifizieren eines Mittels, das zur Behandlung eines mit Fettleibigkeit in Verbindung stehenden medizinischen Zustandes brauchbar ist, wobei das Verfahren die folgenden Schritte umfasst:
(i) Kontaktieren eines Kandidatenmittels mit der menschlichen Site-1-Protease-Promotor-Region, wie in Anspruch 1 definiert; und
(ii) Bestimmen, ob das Kandidatenmittel die Expression des Site-1-Protease-Gens verringert, wobei eine solche Verringerung auf ein Mittel hinweist, das zur Behandlung eines mit Fettleibigkeit in Verbindung stehenden medizinischen Zustandes brauchbar ist.

11. Verfahren zum Identifizieren eines Mittels, das zur Senkung oder Inhibierung der Site-1-Protease-Promotor-Aktivität in der Lage ist, wobei das Verfahren die Überprüfung der Reportergen-Expression in einer Zelle gemäß Anspruch 7 oder 8 in der Gegenwart und Abwesenheit eines Kandidatenmittels umfasst, wobei eine Verringerung oder Inhibierung des Testspiegels der Expression im Vergleich zu einem Kontrollspiegel der Expression auf ein Mittel hinweist, das zur Verringerung oder Inhibierung der Site-1-Protease-Promotor-Aktivität in der Lage ist.

12. Verfahren zum Identifizieren eines Mittels, das zur Behandlung eines mit Fettleibigkeit in Verbindung stehenden medizinischen Zustandes brauchbar ist, wobei das Verfahren die Überprüfung der Reportergen-Expression in einer Zelle gemäß Anspruch 7 oder 8 in der Gegenwart und Abwesenheit eines Kandidatenmittels umfasst, wobei eine Verringerung oder Inhibierung des Testspiegels der Expression im Vergleich zu einem Kontrollspiegel der Expression auf ein Mittel hinweist, das zur Behandlung eines mit Fettleibigkeit in Verbindung stehenden medizinischen Zustandes brauchbar ist.

## Revendications

1. Région promotrice de la protéase de site 1 humaine isolée comprenant une séquence choisie parmi :
(a) la séquence de nucléotides définie comme SEQ ID NO : 2, ou un fragment de celle-ci ayant une activité promotrice de protéase de site 1 ;
(b) le brin complémentaire de (a) ; et
(c) les séquences de nucléotides capables de s'hybrider, sous conditions d'hybridation stringentes, avec une séquence de nucléotides telle que définie en (a) ou (b).

2. Construction recombinante comprenant la région promotrice de la protéase de site 1 humaine selon la revendication 1.

3. Construction recombinante selon la revendication 2 dans laquelle la région promotrice de la protéase de site 1 humaine est opérationnellement liée à un gène codant pour un produit détectable.

4. Construction recombinante selon la revendication 3 dans laquelle ledit gène codant pour un produit détectable est un gène de protéase de site 1 humaine.

5. Construction recombinante selon la revendication 2 comprenant en outre un gène rapporteur.

6. Vecteur comprenant la construction recombinante selon l'une quelconque des revendications 2 à 5.

7. Cellule hôte stablement transformée avec la construction recombinante selon l'une quelconque des revendications 2 à 5.

8. Cellule hôte stablement transformée avec le vecteur selon la revendication 6.

9. Procédé d'identification d'un agent capable de diminuer ou d'inhiber l'activité du promoteur de site 1 de la protéase, ledit procédé comprenant les étapes de :
(i) mettre un agent candidat en contact avec la région promotrice de la protéase de site 1 humaine selon la revendication 1 ; et
(ii) déterminer si ledit agent candidat diminue l'expression du gène de la protéase de site 1, cette diminution indiquant un agent capable de diminuer ou d'inhiber l'activité du promoteur de la protéase de site 1.

10. Procédé d'identification d'un agent utile pour le traitement d'un état médical lié à l'obésité, ledit procédé comprenant les étapes de :
(i) mettre un agent candidat en contact avec la région promotrice de la protéase de site 1 humaine selon la revendication 1 ; et
(ii) déterminer si ledit agent candidat diminue l'expression du gène de protéase de site 1, cette diminution indiquant un agent utile pour le traitement d'un état médical lié à l'obésité.

11. Procédé d'identification d'un agent capable de diminuer ou d'inhiber l'activité du promoteur de la protéase de site 1, ledit procédé comprenant l'analyse de l'expression du gène rapporteur dans une cellule selon la revendication 7 ou 8 en présence et en l'absence d'un agent candidat, dans lequel une diminution ou une inhibition du niveau test d'expression par rapport au niveau témoin d'expression indique un agent capable de diminuer ou d'inhiber l'activité du promoteur de la protéase de site 1.

12. Procédé d'identification d'un agent utile pour le traitement d'états médicaux liés à l'obésité, ledit procédé comprenant l'analyse de l'expression du gène rapporteur dans une cellule selon la revendication 7 ou 8 en présence et en l'absence d'un agent candidat, dans lequel une diminution ou une inhibition du niveau test d'expression par rapport au niveau témoin d'expression indique un agent utile pour le traitement d'états médicaux liés à l'obésité.
